# EUROPÄISCHE PATENTSCHRIFT

(11) **EP 0 694 542 B1**
(45) Veröffentlichungstag und Bekanntmachung des Hinweises auf die Patenterteilung: **03.02.1999**
(21) Anmeldenummer: 95108719.6
(22) Anmeldetag: 07.06.1995
(51) Int. Cl.: C07D 339/04, C07B 55/00

(54) **Verfahren zur Racemisierung von Enantiomeren der alpha-Liponsäure**
Process for racemisation of enantiomers of alpha-lipoic acid
Procédé pour la racémisation des énantiomères de l'acide alpha-lipoique

(30) Priorität: 30.07.1994 DE 4427079
(43) Veröffentlichungstag der Anmeldung: 31.01.1996
(73) Patentinhaber: ASTA Medica Aktiengesellschaft, D-01277 Dresden (DE)
(72) Erfinder: Bethge, Horst, D-63457 Hanau (DE); Möller, Roland, 63546 Hammersbach (DE); Sator, Gerhard, Dr., 64807 Dieburg (DE); Merget, Stefan, 63110 Rodgau (DE); Beisswenger, Thomas, Dr., 01445 Radebeul (DE)
(74) Vertreter: Decker, Karl-Ludwig

(56) Entgegenhaltungen:
- EP-A- 0 261 336
- EP-A- 0 427 247
- EP-A- 0 543 088
- JOURNAL OF THE AMERICAN CHEMICAL SOCIETY, Bd. 78, Dezember 1956 Seiten 6148-6149, R.C. THOMAS ET AL.
- I.L. FINAR 'Organic Chemistry, Volume One: The Fundamental Principles' 1963 , 4. AUFL., LONGMANS , LONDON * Seiten 412-413, insbesondere Seite 412, erster Absatz des Abschnitts "Racemisation" *
- J. FALBE ET AL. (HRSG.) 'Römpp Chemie Lexikon, 9. Aufl., Bd. 5, Pi-S' 1992 , GEORG THIEME VERLAG , STUTTGART - NEW YORK * Seite 3754, Stichwort "Racemisierung" *
- BEYER/WALTER 'Lehrbuch der Organischen Chemie' 1988 , S. HIRZEL VERLAG , STUTTGART * Seite 344, Abschnitt 2.36.3.2 *

## Beschreibung

Die Erfindung betrifft ein Verfahren zur Umwandlung der Enantiomere der α-Liponsäure in das Racemat derselben.α-Liponsäure wird in der Literatur ebenfalls als 1,2-Dithiolan-3-pentansäure oder Thioctsäure bezeichnet.α-Liponsäure besitzt als Coenzym der α-Ketosäuredehydrogenasen in Pflanzen und Tieren eine weite Verbreitung; die natürlich vorkommende Form besitzt die R-Konfiguration. α-Liponsäure ist Pharmakologisch wirksam und weist u.a. antiphlogistische und antinociceptive (analgetische) sowie zytoprotektive Eigenschaften auf. Eine wichtige medizinische Indikation ist die Behandlung der diabetischen Polyneuropathie. Bei den reinen optischen Isomeren der α-Liponsäure (R- und S-Form, d.h. R-α-Liponsäure und S-α-Liponsäure) ist im Gegensatz zu dem Racemat das R-Enantiomere vorwiegend antiphlogistisch und das S-Enantiomere vorwiegend antinociceptiv wirksam (EP 0427247).

Bekannte Synthesen für die reinen optischen Isomeren der α-Liponsäure verlaufen beispielsweise über chirale Vorstufen, die im Laufe der Synthese gespalten werden. Weiterhin kann auch die racemische α-Liponsäure durch Überführung in Salze von reinen optischen Isomeren des α-Methylbenzylamins und Kristallisation getrennt werden, wobei zunächst die reinen diastereomeren Salzpaare isoliert werden (DE-OS 41 37 773.7).
Nachteilig ist bei dem zitierten Verfahren jedoch, daß das jeweils unerwünschte Enantiomer als solches mitunter therapeutisch nicht mehr verwendet werden kann und sich damit das Spaltverfahren für ein gewünschtes reines optisches Isomer der α-Liponsäure unökonomisch gestaltet.

Das mehrstufige Verfahren zur Herstellung enantiomerenreiner R(+)α-Liponsäure und S(-)-α-Liponsäure (EP 0 261 336) vermeidet zwar das Spaltverfahren, bei der technischen Verwertung erweist es sich jedoch ebenfalls als ungünstig.

In I.L. Finar, Organic Chemistry, Volume One: The Fundamental Principles, 4. Auflage, Longmans, London 1963, Seiten 412-413, J. Falbe et al. (Hrsg.), Römpp Chemie Lexikon, 9. Auflage, Bd. 5, Georg Thieme Verlag,Stuttgart 1992, Seite 3754, und Beyer/Walter, Lehrbuch der Organischen Chemie, S. Hirzel Verlag, Stuttgart 1988, Seite 344, wird die Racemisierung von Verbindungen beschrieben, die in Nachbarstellung zu dem chiralen Kohlenstoffatom eine Doppelbindung aufweisen, an der leicht eine Elektronenverschiebung auftreten kann. Im Unterschied dazu sind bei der α-Liponsäure die dem chiralen Zentrum benachbarten Kohlenstoffatome von aliphatischem Charakter; es stehen also keine Elektronen für eine Verschiebung zur Verfügung. Weiterhin ist aus R.C. Thomas, Journal of the American Chemical Society, Bd. 78, 1956, Seiten 6148-6149 bekannt, daß auch bei höheren Temperaturen keine Spaltung an der Kohlenstoff-Schwefel-Bindung der α-Liponsäure auftritt, sondern zwischen den beiden Schwefelatomen. Letztlich ist demnach das mit dem chiralen Zentrum verbundene Wasserstoffatom nicht aktiviert.

Aufgabe der Erfindung ist die Umwandlung von reinen optischen Isomeren der α-Liponsäure oder von Gemischen von reinen optischen Isomeren der α-Liponsäure, bei denen eines der Isomere im Überschuß vorliegt, in das Racemat der α-Liponsäure, um damit das bei der klassischen Racematspaltung zwangsläufig anfallende Enantiomere wieder ökonomisch verwerten zu können.
Eine Racemisierung einer Verbindung ist mit einem Lösen und einer Neubildung von Atombindungen verbunden, die sich bei der α-Liponsäure als schwierig darstellt. α-Liponsäure besitzt eine chemisch und thermisch empfindliche -S-S-Brücke und das Asymmetriezentrum ist weit von der funktionellen - COOH Gruppe entfernt, so daß sterische Wechselwirkungen vermindert sind. Bei einer Racemisierung von α-Liponsäure muß deshalb sowohl unter neutralen Bedingungen als auch unter basisch oder sauer katalysierter Reaktion mit Umlagerungen und Zerfallsprozessen gerechnet werden.

α-Liponsäure (Schmelzpunkt 61°C) und seine reinen optischen Isomere polymerisieren bei Erwärmung rasch und es entsteht eine klebrige zähe polymere α-Liponsäure, die nicht mehr weiterverwendet werden kann. Außerdem schmilzt diese polymere Thioctsäure bei weiterem Erhitzen und zersetzt sich unter Dunkelfärbung. Es konnten nun aber erfindungsgemäß Bedingungen aufgefunden werden, unter denen die Polymerisation einerseits vermieden wird und andernseits eine Zersetzung nicht stattfindet, aber erstaunlicherweise zur Racemisierung der optischen Isomeren Anlaß gibt. Das im Überschuß vorliegende optische Isomer wird dabei racemisiert und die eingesetzte α-Liponsäure liegt schließlich als Racemat vor.

Die vorliegende Aufgabe wurde dadurch gelöst, daß das unerwünschte reine optische Isomer der α-Liponsäure, oder das Gemisch von reinen optischen Isomeren der α-Liponsäure, bei denen eines der Isomere im Überschuß vorliegt, in reiner Form, in Suspension oder in einem geeigneten Lösungsmittel aufgelöst und mit oder ohne Zusatz von Zuschlagstoffen durch Behandlung bei Temperaturen zwischen 110°C bis 200°C unter Normaldruck bis 50 bar während 10 bis 48 Stunden racemisiert. Die Racemisierung wird bevorzugt in einem organischen Lösungsmittel bei einer Temperatur zwischen 150°C und 190°C bei Normaldruck bis 20 bar während 10 bis 48 Stunden durchgeführt, wobei gegebenenfalls noch in einer Menge von 0,01 Mol % bis 5 Mol % saure oder basische Zuschlagsstoffe hinzugegeben werden. Besonders bevorzugt führt man die Racemisierung in organischen Lösungsmitteln mit oder ohne Wasserzusatz bei Temperaturen zwischen 150°C bis 180°C unter einem Druck bis 10 bar während 20 bis 26 Stunden durch. Die Druckbedingungen sind abhängig von der Temperatur, wobei sich bei einer höheren Temperatur ein höherer Druck aufbaut.

Als Lösungsmittel kommen neben Wasser beispielsweise in Frage: aliphatische Kohlenwasserstoffe mit einer Kohlenstoffkettenlänge zwischen 3 und 10, aromatische Kohlenwasserstoffe, die flüssig sind, Ester aus aliphatischen oder cycloaliphatischen Carbonsäuren mit 2 bis 6 Kohlenstoffatomen und aliphatischen oder cycloaliphatischen Alkoholen mit 2 bis 6 Kohlenstoffatomen, Ether und Glycolether oder homogene Mischungen der genannten Lösungsmittel. Besonders bevorzugte Lösungsmittel sind beispielsweise Toluol, Xylol, o-Dichlorbenzol, Essigsäureethylester und Cyclohexan. Geeignete Zuschlagstoffe sind saure oder basische Zusätze zur reinen α-Liponsäure, ihrer Suspension oder Lösung. Neben anorganischen Mineralsäuren und Lewissäuren kommen in Frage: aromatische Sulfonsäuren und Carbonsäuren mit einer Kettenlänge von 1 bis 3. Neben anorganischen Basen kommen in Frage: aliphatische und aromatische Amine und Aminoverbindungen.
Beispielsweise können als Zuschlagsstoffe verwendet werden: Essigsäure, p-Toluolsulfonsäure, Essigsäureester, Triethylamin, Morpholin u.a.
Die racemisierte α-Liponsäure kann direkt oder durch Filtration aus einer Suspension erhalten werden. Die Racemisierungslösungen eignen sich auch, um direkt nach dem zitierten Verfahren (DE-OS 41 37 773.7) eine Kristallisation von reinen optischen diastereomeren Salzpaaren durchzuführen.

Das entstandene Racemat der α-Liponsäure kann anschließend entsprechend der in der DE-OS 41 37 773.7 genannten Methode wieder gespalten werden und liefert weiteres gewünschtes reines optisches Enantiomer. Dadurch wird die gezielte Herstellung eines gewünschten reinen optischen Isomers der α-Liponsäure bedeutend wirtschaftlicher und auch unter ökologischen Gesichtspunkten vorteilhafter. Da nach den gegenwärtigen Erkenntnissen das R(+) Enantiomere pharmakologisch bedeutsamere Wirkungen zeigt, wurde bei den Racemisierungen vorwiegend das S(-)-Enantiomere oder Gemische der optischen Isomeren mit einem Überschuß des S(-)-Enantiomeren eingesetzt. In gleicher Weise läßt sich natürlich das R(+)-Enantiomere oder Gemische der optischen Isomeren mit einem Überschuß des R(+)-Enantiomeren racemisieren. Die Ausbeuten bei der Racemisierung betragen je nach den Reaktionsbedingungen ca. 70 % mit einem racemisierten Anteil eines Enantiomeren bis 49 %.

Die Reinheit der optischen Isomere wurde mittels der spezifischen optischen Drehwerte bestimmt.

Weiterhin wurden durch HPLC an optisch aktiven Säulen relative Gehalte der optischen Isomere der α-Liponsäure mit einer Nachweisgrenze von > 0,5 % bestimmt. Hierzu wurde eine homogene Probe des jeweiligen Reaktionsansatzes eingesetzt.

Die vorliegende Erfindung wird durch nachfolgende Beispiele näher erläutert.

### Beispiel 1

75 g (S)-(-)-α-Liponsäure wurden unter Stickstoff bei 150°C gerührt. Nach 22 h wurde abgekühlt. Der Gehalt an R-(+)-α-Liponsäure betrug 3 %.

### Beispiel 2

75 g S-(-)-α-Liponsäure wurden unter Stickstoff bei 160°C gerührt. Nach 22 h wurde abgekühlt. Der Gehalt an R-(+)-α-Liponsäure betrug 15,3 %.

### Beispiel 3

75 g S-(-)-α-Liponsäure wurden unter Stickstoff bei 170°C gerührt. Nach 22 h wurde abgekühlt. Der Gehalt an R-(+)-α-Liponsäure betrug 47,8 %.

Die Ausbeuten der Racemisierungen in fester Form vermindern sich jedoch aufgrund der ZerSetzungsgeschwindigkeiten der α-Liponsäure ab 160°C.

### Beispiel 4

- Variante:: Lösungsmittel: Toluol
Temperatur: 180°C
Eigendruck: 6 bar

75 g S-(-)-α-Liponsäure werden in 300 ml Toluol gelöst (klare gelbe Lösung). Diese Lösung wird in einen 1000 ml-Autoklav eingefüllt. Nach dem Verschließen des Autoklaven wird auf 180°C angeheizt und 24 Stunden bei dieser Temperatur gerührt. Hierbei stellt sich ein Eigendruck von ca. 6 bar ein.
Nach beendeter Reaktionszeit wird die Lösung aus dem Autoklaven entnommen und das Toluol im Vakuum bei einer Sumpftemperatur von 40-50°C abdestilliert.
Der zurückbleibende ölige Sumpf wird mit 500 ml eines Gemisches bestehend aus 333 ml Cyclohexan/167 ml Essigester versetzt und auf 40-50°C angewärmt. Nach kurzer Zeit erhält man eine klare gelbe Lösung.
Die Lösung wird mit 1 g Diacel® Cellulose-Filterhilfsmittel der Firma Cellulose-Füllstoff-Vertriebs GmbH Mönchengladbach versetzt und 5 Min. gerührt.
Anschließend wird über eine Nutsche abgesaugt und mit 50 ml Cyclohexan/Essigester-Gemisch nachgewaschen. Das Filtrat wird langsam unter Rühren abgekühlt. Bei ca. 5°C beginnt die Kristallisation der α-Liponsäure. Nachdem die α-Liponsäure auskristallisiert ist, wird weiter bis auf ca. - 5°C abgekühlt und 2 Stunden bei dieser Temperatur nachgerührt. Anschließend wird das Produkt über eine Nutsche abgesaugt und mit 50 ml Cyclohexan nachgewaschen. Die lösungsmittelfeuchte α-Liponsäure wird bei 20-25°C im Vakuum (10-20 mmbar) getrocknet.

Man erhält 57 g α-Liponsäure α_{D}²⁰ = 0° (c = 1, Ethanol) Dies entspricht einer Ausbeute von 76 %

Der Anteil von R(+)α-Liponsäure beträgt 49 %

Die in der Mutterlauge verbleibende α-Liponsäure kann teilweise durch Kristallisation aus der auf 1/3-eingeengten Mutterlauge recycliert werden. Dieses Zweitkristallisat muß einer weiteren Umkristallisation unterzogen werden.

Weitere Racemisierungen wurden unter folgenden Bedingungen durchgeführt

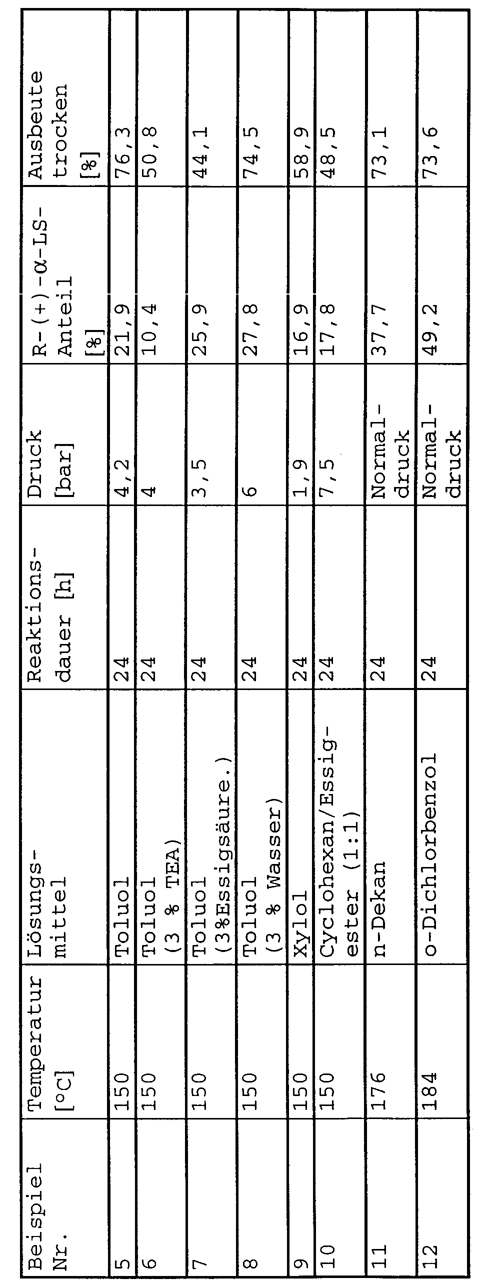

Die relativen Gehalte der optischen Isomere der α-Liponsäure wurden durch HPLC an optisch aktiven Säulen bestimmt

### Enantiomerentrennung von R,S-Thioctsäure an chiralem Trägermaterial

### Chromatographische Bedingungen

- Säule: : Chiral AGP (Fa. ICT)
- Säulendimension: : 100 mm x 4 mm
- Vorsäule: : Chiral AGP (Fa. ICT)
- Säulendimension: : 10 mm x 3 mm
- Mobile Phase: : 1000 ml 0,01 m di-Natriumhydrogenphosphatlösung mit verd. Phosphorsäure auf einen pH-Wert von 4.95-5.05 einstellen, 150 ml Methanol zugeben und durchmischen.
- Flußrate: : 0.5 ml/min
- Druck: : ca. 35 bar
- Temperatur: : 20°C
- Wellenlänge: : 210 nm

### Probenvorbereitung

Etwa 1.5-2 mg des jeweiligen Antipoden werden mit 1 ml Methanol gelöst und mit mobiler Phase auf 20 ml verdünnt. Bei Bedarf ist die Lösung über ein 0.45 µm Membranfilter zu filtrieren. 20 µl dieser Lösung werden injiziert.

## Patentansprüche

1. Verfahren zur Racemisierung von reinen optischen Isomeren der α-Liponsäure oder Gemischen von reinen optischen Isomeren der α-Liponsäure, bei denen eines der optischen Isomere im Überschuß vorliegt,
dadurch gekennzeichnet,
daß die α-Liponsäure in fester Form unter Stickstoff oder in Suspension bei Temperaturen zwischen 150°C und 200°C unter Normaldruck bis 50 bar während 10 bis 48 Stunden racemisiert wird oder in einem inerten organischen Lösungsmittel-Gemisch mit oder ohne Wasserzusatz, gegebenenfalls unter Zusatz basisch oder sauer reagierender Hilfsstoffe, bei Temperaturen zwischen 110°C und 200°C unter Normaldruck bis 50 bar während 10 bis 48 Stunden racemisiert wird.

2. Verfahren nach Anspruch 1,
dadurch gekennzeichnet,
daß die Racemisierung in flüssiger Form durchgeführt wird und basisch oder sauer reagierende Hilfsstoffe in einem Verhältnis von 0,01 Mol % bis 5 Mol % zugegeben werden.

3. Verfahren nach Anspruch 1,
dadurch gekennzeichnet,
daß die Racemisierung bei einer Temperatur zwischen 150°C und 190°C durchgeführt wird.

4. Verfahren nach Anspruch 1,
dadurch gekennzeichnet,
daß die Racemisierung bei Normaldruck bis 20 bar durchgeführt wird.

5. Verfahren nach Anspruch 1,
dadurch gekennzeichnet,
daß die Reaktionszeit der Racemisierung zwischen 20 bis 40 Stunden beträgt.

6. Verfahren nach Anspruch 1,
dadurch gekennzeichnet,
daß die Racemisierung in aromatischen oder aliphatischen organischen Lösungsmitteln durchgeführt wird.

7. Verfahren nach Anspruch 2,
dadurch gekennzeichnet,
daß die Hilfsstoffe ausgewählt sind aus der Gruppe Essigsäure, p-Toluolsulfonsäure, Essigsäureester, Triethylamin, Morpholin.

## Claims

1. Process for the racemization of pure optical isomers of α-lipoic acid or mixtures of pure optical isomers of α-lipoic acid where one of the optical isomers is present in excess,
characterized in that
the α-lipoic acid is racemized in solid form under nitrogen or in suspension at temperatures between 150°C and 200°C under from atmospheric pressure to 50 bar over 10 to 48 hours, or is racemized in an inert organic solvent mixture with or without addition of water, if appropriate with addition of basic or acidic auxiliaries, at temperatures between 110°C and 200°C under from atmospheric pressure to 50 bar over 10 to 48 hours.

2. Process according to Claim 1,
characterized in that
the racemization is carried out in liquid form and basic or acidic auxiliaries are added in a ratio of from 0.01 mol% to 5 mol%.

3. Process according to Claim 1,
characterized in that
the racemization is carried out at a temperature between 150°C and 190°C.

4. Process according to Claim 1,
characterized in that
the racemization is carried out at from atmospheric pressure to 20 bar.

5. Process according to Claim 1,
characterized in that
the reaction time of the racemization is between 20 and 40 hours.

6. Process according to Claim 1,
characterized in that
the racemization is carried out in aromatic or aliphatic organic solvents.

7. Process according to Claim 2,
characterized in that
the auxiliaries are selected from the group consisting of acetic acid, p-toluenesulphonic acid, ethyl acetate, triethylamine, morpholine.

## Revendications

1. Procédé de racémisation d'isomères optiquement purs de l'acide α-lipoïque ou de mélanges d'isomères optiquement purs de l'acide α-lipoïque, dans lequel l'un des isomères optiques se présente en excès,
caractérisé en ce qu'
on racémise l'acide α-lipoïque sous forme pure sous azote ou en suspension à des températures comprises entre 150°C et 200°C sous une pression normale allant jusqu'à 50 bars pendant 10 à 48 heures ou dans un mélange de solvants organiques inertes avec ou sans addition d'eau, le cas échéant avec addition d'additifs à réaction basique ou acide, à des températures comprises entre 110°C et 210°C à une pression normale allant jusqu'à 50 bars pendant 10 à 48 heures.

2. Procédé selon la revendication 1,
caractérisé en ce qu'
on effectue la racémisation sous forme liquide et en ce qu'on ajoute des adjuvants à réaction basique ou acide dans un rapport de 0,01 % molaire à 5 % molaire.

3. Procédé selon la revendication 1,
caractérisé en ce qu'
on effectue la racémisation à une température comprise entre 150°C et 190°C.

4. Procédé selon la revendication 1,
caractérisé en ce qu'
on effectue la racémisation à pression normale allant jusqu'à 20 bars.

5. Procédé selon la revendication 1,
caractérisé en ce que
le temps de réaction de la racémisation est compris entre 20 et 40 heures.

6. Procédé selon la revendication 1,
caractérisé en ce qu'
on effectue la racémisation dans des solvants organiques aromatiques ou aliphatiques.

7. Procédé selon la revendication 2,
caractérisé en ce que
les adjuvants sont choisis dans le groupe constitué par l'acide acétique, l'acide p-toluènesulfonique, l'acétate d'éthyle, la triéthylamine, la morpholine.
